# EUROPÄISCHE PATENTSCHRIFT

(11) **EP 1 002 550 B1**
(45) Veröffentlichungstag und Bekanntmachung des Hinweises auf die Patenterteilung: **25.05.2005**
(21) Anmeldenummer: 99120709.3
(22) Anmeldetag: 19.10.1999
(51) Int. Cl.: A61M 1/16

(54) **Kartuschenhalter für eine Dialysemaschine**
Cartridge holder for a dialysis apparatus
Porte-cartouche pour un appareil de dialyse

(30) Priorität: 17.11.1998 DE 19852982
(43) Veröffentlichungstag der Anmeldung: 24.05.2000
(73) Patentinhaber: B. Braun Medizintechnologie GmbH, 34212 Melsungen (DE)
(72) Erfinder: Bock, Gerhard, 36289 Friedewald (DE); Otto, Reinhold, 34323 Malsfeld (DE); Mardorf, Robert, 34212 Melsungen (DE)
(74) Vertreter: Selting, Günther, Dipl.-Ing.

(56) Entgegenhaltungen:
- EP-B- 0 278 100
- WO-A-97/02056

## Beschreibung

Die Erfindung betrifft einen Kartuschenhalter für eine Dialysemaschine, in welcher die Herstellung der Dialysierflüssigkeit durch Auflösen eines in einer Kartusche befindlichen Salzes oder durch Verdünnen eines flüssigen Konzentrats erfolgt.

In Dialysemaschinen wird eine Dialysierflüssigkeit aufbereitet, die durch einen Dialysator geleitet wird, um dem Blut des Patienten Schadstoffe zu entziehen. Außerdem wird das Dialysegerät vor einer Behandlung desinfiziert. Aus EP 0 278 100 B1 ist es bekannt, die Dialysierflüssigkeit durch Lösen eines Pulvers in Wasser in der Dialysemaschine zu erzeugen, indem eine das Pulver enthaltende Kartusche von Wasser durchströmt wird. Bei dem Pulver handelt es sich in der Regel um Natriumbicarbonat. Auf diese Weise wird ein Flüssigkeitskonzentrat erzeugt, das später noch mit Wasser verdünnt wird, um die für die Dialyse erforderliche Konzentration zu erhalten.

Aus WO 97/02056 ist ein Kartuschenhalter für eine Dialysemaschine bekannt, bei der die Kartusche zwischen zwei Backen eingesetzt wird, welche relativ zueinander längs einer Linearführung verschiebbar sind. Beim Einsetzen der Kartusche zwischen die Backen dringen an den Backen vorgesehene Dorne in die Kartusche ein und nebenfalls an den Backen vorgesehene Anschlüsse stellen eine Fluidverbindung mit dem Kartuscheninneren her. Auf diese Weise kann durch den oberen Backen Wasser zugeführt werden, während aus dem unteren Backen die Salz-Wasser-Lösung abgeführt wird. Für eine Entsalzung, Reinigung oder Desinfizierung ist es erforderlich, die flüssigkeitsführenden Teile des Kartuschenhalters zu durchspülen. Hierzu können die Backen mit ihren Anschlüssen unmittelbar gegeneinandergesetzt werden, so daß sie einen Kurzschluß bilden, durch den eine Spül- oder Desinfektionsflüssigkeit geleitet wird. Dabei werden die flüssigkeitsführenden Teile und auch die Dichtungen durchspült. Zur Ermöglichung des Kurzschlusses haben die beiden Backen einen komplizierten Aufbau mit zahlreichen Dichtungselementen. Die Anschlüsse der beiden Backen sind grundsätzlich von gleicher Anschlußart, d.h. sie weisen eine Ausnehmung zum abdichtenden Einschiebern eines Ansatzes der Kartusche sowie einen Dorn auf. Dies hat zur Folge, daß die Anschlüsse des oberen und des unteren Backens nicht unmittelbar zusammenpassen. Daher müssen diese Anschlüsse durch übergreifende Zusatz-Anschlußelemente so modifiziert werden, daß sie abdichtend gegeneinandergesetzt werden können. Dadurch erhalten die beiden Anschlüsse einen komplexen Aufbau.

Der Erfindung liegt die Aufgabe zugrunde, einen Kartuschenhalter zu schaffen, der es ermöglicht, die beiden Backen miteinander zu verbinden oder kurzzuschließen, ohne daß diese Backen hierfür modifiziert werden müßten.

Bei dem erfindungsgemäßen Kartuschenhalter ist ein Zwischenbacken oder Adapter vorgesehen, der zwischen den unteren und den oberen Backen einsetzbar ist, um die Anschlüsse dieser Backen zu verbinden. Ein wesentlicher Vorteil besteht darin, daß die Anschlüsse des unteren und des oberen Backens gleichartig sein können, da sie nicht direkt zusammengesteckt werden. An dem Zwischenbacken befinden sich die entsprechenden Gegenanschlußelemente, die mit dem jeweiligen Anschluß des unteren und des oberen Backens zusammenpassen. Auf diese Weise können diejenigen Anschlüsse, die für die Verbindung mit der Kartusche benutzt werden, auch für die Verbindung mit dem Zwischenbacken benutzt werden, so daß keine zusätzlichen Dichtungselemente, die der Gefahr der Salzablagerung oder Verkrustung ausgesetzt sind, benötigt werden. Die Gegenanschlußelemente des Zwischen-Backens können leicht gereinigt und desinfiziert werden, da sie bei inaktivem Zwischenbacken gut zugänglich sind.

Gemäß einer bevorzugten Ausführungsform der Erfindung ist der Zwischenbacken um eine parallel zur Verschieberichtung des oberen oder unteren Backens verlaufende Achse schwenkbar. Der Zwischenbacken wird längs derselben Stange verschoben, an der auch der obere oder der untere Backen verschoben wird. Zusätzlich kann er um diese Stange herum verschwenkt werden. Der Zwischenbacken ist unverlierbarer Bestandteil der vertikalen Führung, an der der bewegbare Backen gleitet. Er kann um die Achse dieser Führung herum verschwenkt werden.

Vorzugsweise ist der Zwischenbacken in Abhängigkeit von der Höhenposition des oberen Backens um eine vertikale Achse schwenkbar, wobei er dann in die Ebene der beiden äußeren Backen gelangt, wenn der obere Backen unter eine Mindesthöhe absinkt. Diese Mindesthöhe ist niedriger als die Höhe, die der obere Backen bei eingesetzter Kartusche einnimmt. Wenn der obere Backen diese Mindesthöhe unterschreitet, wird der Zwischenbacken automatisch in die Ebene der beiden.äußeren Backen eingeschwenkt, so daß die äußeren Backen nicht in gegenseitigen Kontakt kommen können, weil sich zuvor der Zwischenbacken dazwischenschiebt. Diese Steuerung des Zwischenbackens kann durch eine Folgesteuerung, Kulissensteuerung oder Nokensteuerung erfolgen.

Grundsätzlich besteht die Möglichkeit, den bewegbaren Backen, bei dem es sich in der Regel um den oberen Backen handelt, manuell entlang der Führungsschiene zu verschieben. Durch die manuelle Verschiebung des oberen Backens wird auch die Kraft zum Verschwenken des Zwischenbackens aufgebracht. Eine andere Lösung sieht vor, daß der verschiebbare Backen automatisch mit einer Spindel oder einer anderen Kraftübertragungsvorrichtung verschoben wird. Hierbei erfolgt das Bewegen des oberen Backens durch automatische Steuerung und/oder durch manuelles Betätigen eines Schalters. Die Schwenkbewegung des Zwischenbackens ist vorzugsweise durch den bewegbaren Backen über eine Nockensteuerung gesteuert.

Der Zwischenbacken wird derart bewegt, daß er nach dem Einschwenken in die Ebene der äußeren Backen auf den unteren Backen abgesenkt wird. Dadurch ist es möglich, das Anschlußelement des Zwischenbackens mit dem Anschluß des unteren Backens in gegenseitiger Ausrichtung zusammengreifen zu lassen. Beim nachfolgenden Auseinanderbewegen wird der Zwischenbacken zunächst linear angehoben und erst verschwenkt, nachdem sein Anschlußelement von dem Anschluß des unteren Backens gelöst worden ist.

Es besteht die Möglichkeit, den Zwischenbacken nur als Adapter zu benutzen, der die gleichartigen Anschlüsse der beiden äußeren Backen untereinander verbindet, wenn sich keine Kartusche zwischen ihnen befindet. Andererseits besteht auch die Möglichkeit, den Zwischenbacken für die Desinfektion des Halters in der Flüssigkeitsleitung zu benutzen. Außerdem kann der Zwischenbacken ein Reservoir enthalten, in dem das Desinfektionsmittel als lösliches Pulver oder Konzentrat enthalten ist. Während der Desinfektion wird in dem vorhandenen Flüssigkeitsweg das Pulver mittels Wasser gelöst und verdünnt in die Flüssigkeitswege der Dialysemaschine zur Desinfektion eingebracht. Das Desinfektionsmittel kann in dem Zwischenbacken auch als flüssiges Konzentrat enthalten sein, das im Flüssigkeitsweg mit Wasser verdünnt wird. So kann der Zwischenbacken ein Reservoir enthalten, in dem das Spül- oder Desinfektionsmittel als lösliches Pulver enthalten ist. Hierbei braucht lediglich Wasser zugeführt zu werden, um eine für die Gerätebehandlung erforderliche Lösung in Form eines Konzentrats zu erhalten. Die Größe des Zwischenbackens kann so groß sein wie die zur Herstellung der Dialysierflüssigkeit benutzten Kartuschen.

Gemäß einer besonders zweckmäßigen Ausführungsform der Erfindung weist der Zwischenbacken einen Kartuschenträger auf und er ist derart schwenkbar, daß in der einen Position die Anschlußelemente und in der anderen Position der Kartuschenträger zu den Anschlüssen des unteren und des oberen Backens ausgerichtet sind. Hierbei kann der Kartuschenträger während des Spülens in eine inaktive Position geschwenkt und mit einer neuen Kartusche beschickt werden. Nach Beendigung des Spülens wird der Kartuschenträger mit der neuen Kartusche automatisch in die Betriebsposition geschwenkt. Wenn der in einer Kartusche enthaltene Pulvervorrat verbraucht ist, kann diese Kartusche aus der Ausrichtung mit den beiden Backen herausgeschwenkt werden, damit sie außerhalb der Betriebsposition von Wasser leerlaufen kann. Außerdem ist es möglich, ein automatisches Auswerfen der Kartusche und die Zuführung neuer Kartuschen durchzuführen.

Im folgenden werden unter Bezugnahme auf die Zeichnungen Ausführungsbeispiele der Erfindung näher erläutert.

Es zeigen:
- Fig. 1: eine perspektivische Ansicht des Kartuschenhalters in der Betriebsposition mit eingesetzter Kartusche,
- Fig. 2: die Darstellung eines Kartuschenwechsels,
- Fig. 3: das Herunterfahren des oberen Backens und das Einschwenken des Zwischenbackens,
- Fig. 4: die drei Backen im verbundenen Zustand während des Spülens oder Desinfizierens,
- Fig. 5: eine Ansicht von Fig. 2 aus Richtung des Pfeiles V,
- Fig. 6: eine Ansicht aus Richtung der Pfeile VI-VI von Fig. 5,
- Fig. 7: einen Schnitt entlang der Linie VII-VII von Fig. 5,
- Fig. 8: in gleicher Darstellung wie Fig. 6 den Zustand des Absenkens des oberen Backens und des Einschwenkens des Zwischenbackens,
- Fig. 9: in gleicher Darstellung wie Fig. 8 alle drei Backen in miteinander verbundenem Zustand,
- Fig. 10: in gleicher Darstellung wie Fig. 7 den ausgerichteten Zustand aller drei Backen nach Fig. 9,
- Fig. 11: ein zweites Ausführungsbeispiel des Kartuschenhalters mit eingesetzter Kartusche, die von einem. Kartuschenträger gehalten wird,
- Fig. 12: das Einschwenken des Zwischenbackens unter gleichzeitigem Ausschwenken des Kartuschenträgers,
- Fig. 13: den Spülzustand mit Auswechslung der Kartusche,
- Fig. 14: eine Ansicht von Fig. 11 aus Richtung des Pfeiles XIV,
- Fig. 15: in gleicher Darstellung wie Fig. 14 das Hochfahren des oberen Backens,
- Fig. 16: einen Schnitt entlang der Linie XVI-XVI von Fig. 15,
- Fig. 17: in gleicher Darstellung wie Fig. 16 das Verschwenken des Zwischenbackens,
- Fig. 18: einen Schnitt entlang der Linie XVIII-XVIII von Fig. 15 und
- Fig. 19: in gleicher Ansicht wie Fig. 18 die Position des Zwischenbackens beim Spülbetrieb.

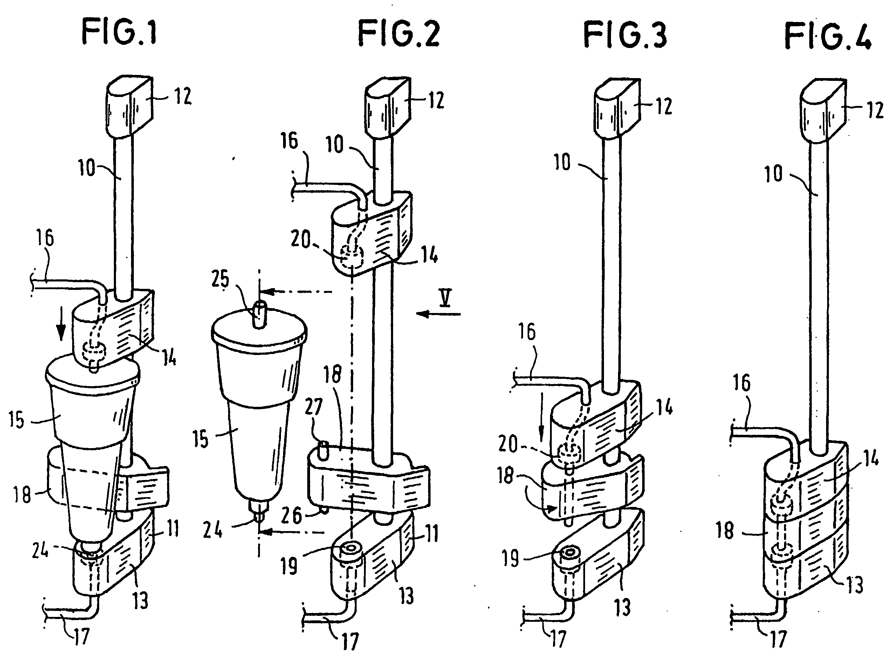

Bei dem Ausführungsbeispiel der Fign. 1-10 ist eine vertikale Führungsstange 10 vorgesehen, die an der Vorderfront einer (nicht dargestellten) Dialysemaschine angebracht sein kann. Die Führungsstange 10 ist an ihrem unteren Ende mit einem Halter 11 und an ihrem oberen Ende mit einem Halter 12 versehen, die an der Dialysemaschine befestigt sind. An dem unteren Halter 11 ist der untere Backen 13 in Form eines schräg nach vorne abstehenden Auslegers befestigt. Längs der Führungsstange 10 ist ein oberer Backen 14 bewegbar. Der obere Backen 14 ist unverdrehbar an der Führungsstange geführt und er ist deckungsgleich mit dem unteren Backen 13. Gemäß Fig. 1 kann eine Kartusche 15, die pulveriges Bicarbonat-Konzentrat enthält, auf den unteren Backen 13 aufgesetzt werden, wonach der obere Backen 14 heruntergedrückt wird und mit dem Oberteil der Kartusche 15 zusammenwirkt. In den oberen Backen 14 führt eine Zulaufleitung 16 hinein und aus dem unteren Backen 13 führt eine Ablaufleitung 17 heraus.

Zwischen den beiden Backen 13,14 ist ein Zwischenbacken 18 angeordnet, der ebenfalls an der Führungsstange 10 geführt ist. Im Gegensatz zu dem oberen Backen 14 kann der Zwischenbacken 18 jedoch um die Führungsstange 10 verschwenkt werden.

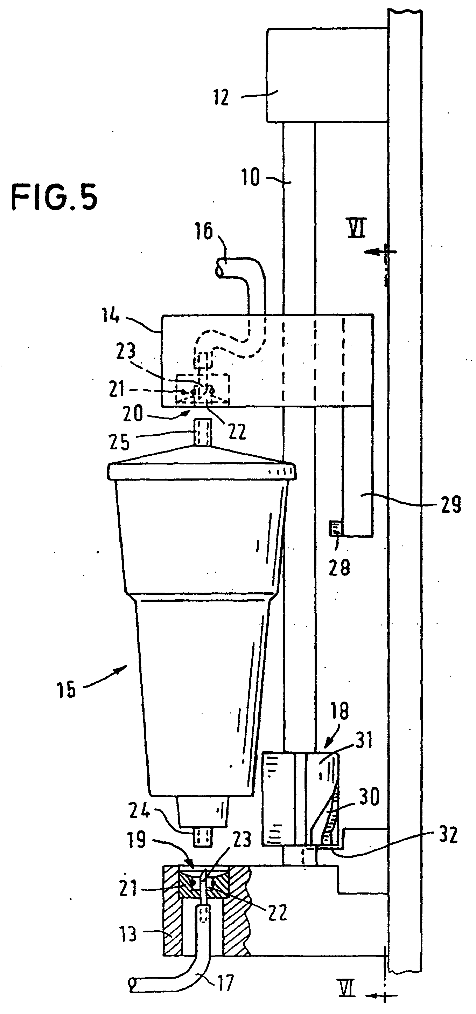

Jeder der Backen 13 und 14 weist einen Fluidanschluß 19 bzw. 20 auf. Jeder Fluidanschluß besteht aus einem Aufnahmeteil 21 (Fig. 5) mit einer zentralen Vertiefung 22 und einem vom Boden der Vertiefung abstehenden Dorn 23.

Die Kartusche 15 hat an ihrem unteren Ende einen zylindrischen Nippel 24 und an ihrem oberen Ende einen zylindrischen Nippel 25. Der Nippel 24 wird in die Vertiefung 22 passend eingedrückt, wobei der Dorn 23 die Stirnwand des Nippels 24 durchdringt und die Verbindung der Abflußleitung 17 mit dem Innern der Kartusche herstellt. In gleicher Weise wird der obere Nippel 25 abdichtend in die Vertiefung 22 des oberen Anschlusses 20 eingeführt, wobei der Dorn 23 die Stirnwand des Nippels 25 durchdringt und die Verbindung des Kartuscheninnern mit der Zulaufleitung 16 herstellt.

Wenn die Kartusche 15 gemäß Fig. 1 auf den unteren Backen 13 aufgestellt wurde, wobei der Nippel 24 abdichtend in der Vertiefung 22 des Anschlusses 19 aufgenommen wird, wird der obere Backen 14 in Richtung auf die Kartusche nach unten verschoben, bis der Dorn des Anschlusses 20 die Kartuschenwand durchdringt. Mit den Backen 13,14 wird die Kartusche 15 in vertikalem Zustand sicher festgehalten und über die Zulaufleitung 16 wird Wasser zugeführt, in welchem sich das in der Kartusche 15 enthaltene Salz löst. Die Zulaufleitung 16 enthält ein Absperrventil, das in Abhängigkeit von der durch Sensoren festgestellten Position des Zwischenbackens 18 und des oberen Backens 14 öffnet. Durch die Leitung 17 wird die Lösung abgeführt.

In Fig. 2 ist das Entnehmen der Kartusche 15 dargestellt. Der obere Backen 14 wird mit der Hand hochgeschoben, so daß die Kartusche 15 freikommt und nach geringfügigem Anheben seitlich entnommen werden kann.

Um das Leitungssystem des Kartuschenhalters zu desinfizieren und zu spülen, wird der obere Backen 14 aus der in Fig. 2 dargestellten Position nach unten geschoben, wie dies in Fig. 3 dargestellt ist. Hierbei wird automatisch der Zwischenbacken 18 aus der in Fig. 2 dargestellten Bereitschaftsposition in die Betriebsposition geschwenkt. Das Schwenken erfolgt automatisch durch einen Mitnehmer, sobald die Höhe des oberen Backens 14 eine bestimmte Mindesthöhe, die kleiner ist als die in Fig. 1 dargestellte Arbeitshöhe, unterschritten hat. Durch die Kraft der Abwärtsbewegung des oberen Backens 14 wird der Zwischenbacken 18 so verschwenkt, daß er mit den äußeren Backen 13,14 zur Ausrichtung kommt. Diese Ausrichtung ist erreicht, bevor die drei Backen sich gegenseitig berühren. Anschließend wird durch weiteres Niederdrücken des oberen Backens 14 der Zwischenbacken 18 mit den äußeren Backen 13 und 14 verbunden. Wenn der Zwischenbacken 18 gemäß Fig. 4 zu den äußeren Backen 13,14 ausgerichtet und mit seinen Anschlußelementen 26,27 in die Anschlüsse 19,20 abdichtend eingesetzt ist, sind die Zulaufleitung 16 und die Ablaufleitung 17 kurzgeschlossen und das so gebildete Leitungssystem kann von einem Spül- und/oder Desinfektionsmittel durchströmt werden. Der Ausrichtzustand des Zwischenbackens 18 gemäß Fig. 4 wird durch einen im unteren Backen befindlichen Sensor festgestellt, um die Spülung erst im korrekt ausgerichteten Zustand der Backen einzuleiten.

Nach erfolgter Spülung wird der obere Backen 14 hochgeschoben, wobei der Zwischenbacken 18 zunächst parallel angehoben und anschließend um die Führungsschiene 10 herum nach außen geschwenkt wird. Nachdem die in Fig. 2 dargestellte Position erreicht ist, kann eine neue Kartusche 15 eingeführt und durch Niederdrücken des oberen Backens 14 fixiert werden.

Die Steuerung der Schwenkbewegung des Zwischenbackens 18 erfolgt durch einen Nocken 28, der an einem von dem oberen Backen 14 nach unten ragenden Arm 29 befestigt ist. Die vertikale Länge des Armes 29 überragt die Höhe des Zwischenbackens 18.

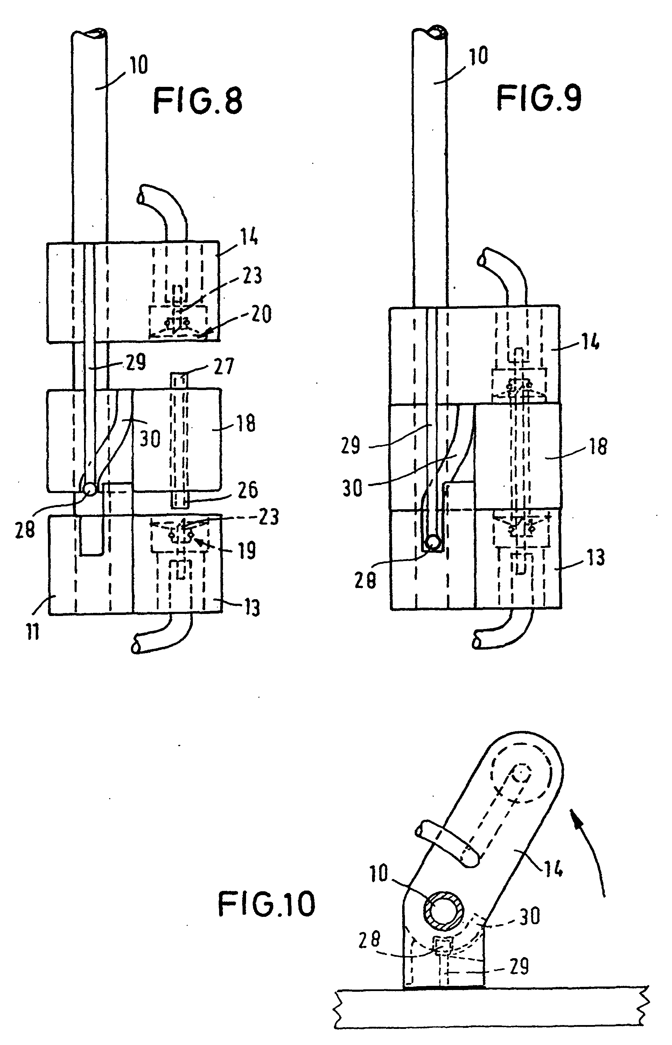

Der Nocken 28 wirkt mit einer Führungsnut 30 zusammen, die in einem zylindrischen Bereich 31 an der Rückseite des Zwischenbackens 18 vorgesehen ist. Der Zwischenbacken 18 kann in vertikaler Richtung in Grenzen frei bewegt werden, nämlich von der in den Fign. 4 und 9 dargestellten Aufsetzposition in die in den Fign. 3 und 8 dargestellte Hubposition, in der das hervorstehende untere Anschlußelement 26 gerade von dem unteren Backen 13 freikommt. Die Aufsetzposition kann nur eingenommen werden, wenn der Zwischenbacken 18 exakt zu dem unteren Backen 13 ausgerichtet ist.

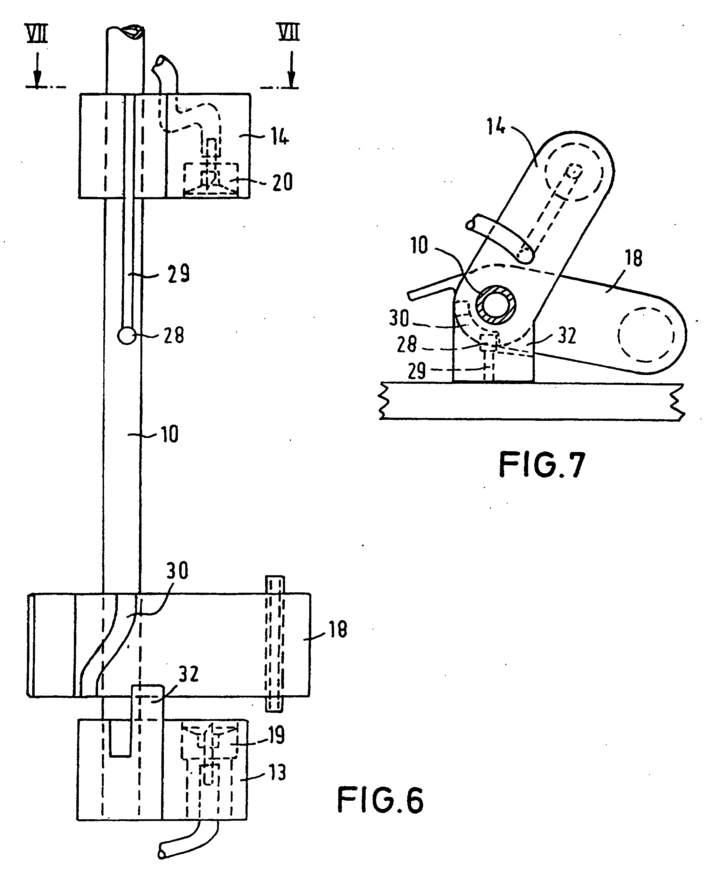

Beim Absenken des oberen Backens 14 greift nach Unterschreiten der Mindesthöhe der Nocken 28 in das obere Ende der Führungsnut 30 des Zwischenbackens 18 ein (Fign. 5 und 6), welcher sich in der Ausschwenkposition befindet. Die Führungsnut 30 hat eine Schrägflanke, so daß der Zwischenbacken 18 um die Achse der Führungsschiene 10 geschwenkt wird, wenn der Nocken 30 senkrecht nach unten durch die Führungsnut 30 hindurchbewegt wird.

In der Hubposition im ausgeschwenkten Zustand ruht der Zwischenbacken 18 auf einer Auflagefläche 32 des Halters 11. Aus der Hubposition kann der Zwischenbacken nur absinken, wenn er zuvor in Ausrichtung mit den äußeren Backen 13,14 gebracht wurde. Im Zuge der Abwärtsbewegung des oberen Backens verschwenkt dieser zunächst durch Eingreifen des Nockens 28 in die Führungsnut 30 den Zwischenbacken 18 zur Seite und anschließend sinkt der Zwischenbacken 18 aufgrund seines Gewichts auf den unteren Backen 13, wobei das Anschlußelement 26 in den Anschluß 19 eindringt. Beim weiteren Niederdrücken des oberen Backens 14 setzt sich dieser voll gegen den Zwischenbacken 18, wobei dessen Anschlußelement 27 in den Anschluß 20 eindringt.

Die Anschlüsse 19 und 20 sind untereinander gleich ausgebildet. Im vorliegenden Fall sind es weibliche Anschlüsse, während die Anschlußelemente 26,27 des Zwischenbackens 18 männlich sind.

Beim Hochziehen des oberen Backens 14 löst dieser sich . zunächst von dem Zwischenbacken 18. Dann nimmt er den Zwischenbacken 18 ein Stück weit senkrecht nach oben mit und schließlich verschwenkt er den Zwischenbacken 18 durch die Wirkung des in den schrägen Teil der Führungsnut 29 eingreifenden Nockens 28.

Abweichend von dem beschriebenen Ausführungsbeispiel kann die Vorrichtung auch motorisch betrieben werden, wobei der obere Backen 14, beispielsweise von einer Spindel, in vertikaler Richtung angetrieben werden kann.

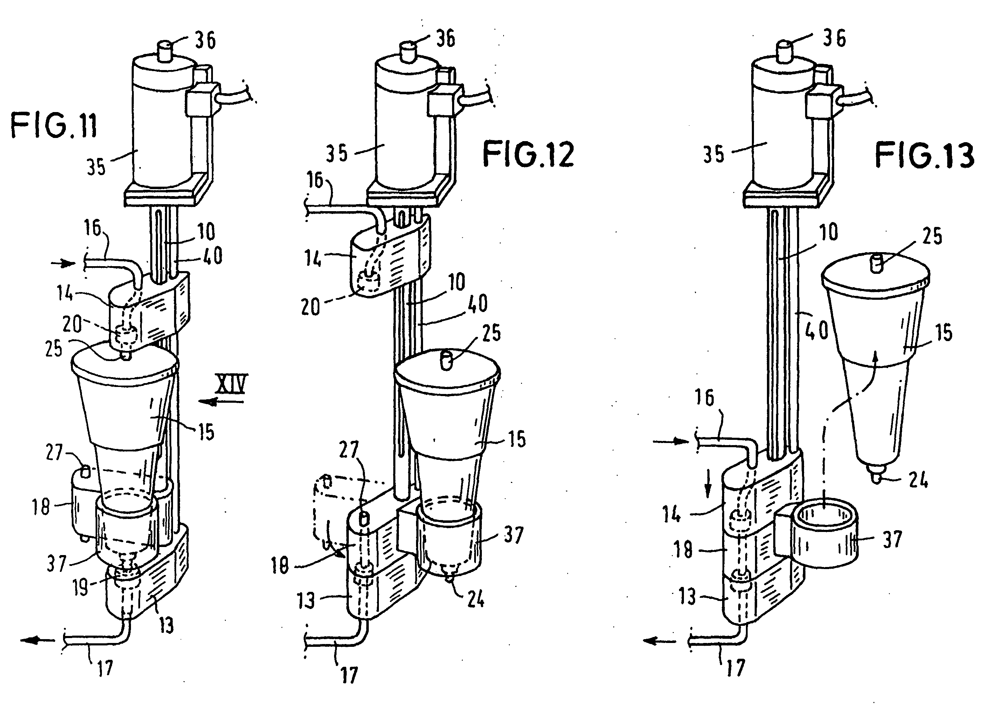

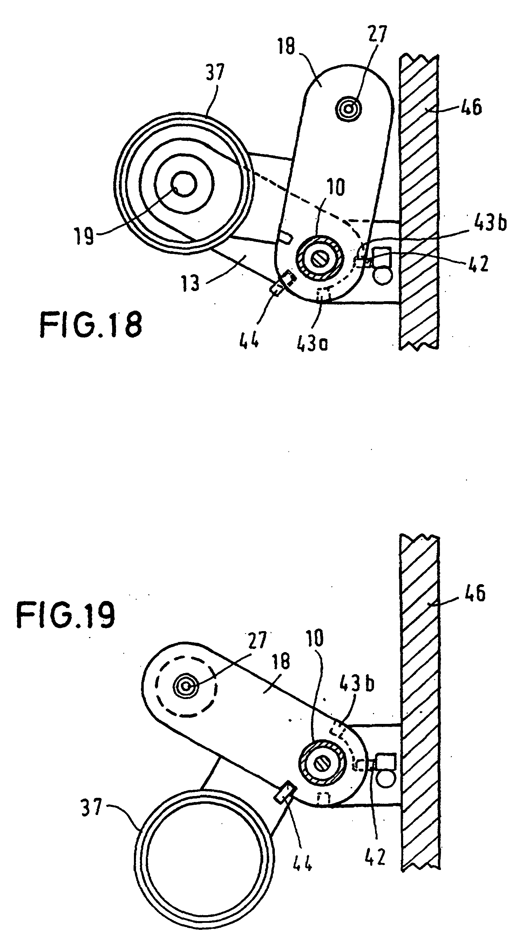

Eine modifizierte Motorlösung ist in den Fign. 11-19 dargestellt. Hierbei ist der untere Backen 13 ebenfalls an einer Führungsschiene 10 fest angebracht, während der obere Backen 14 längs der Führungsschiene 10 verfahrbar und gegen Verschwenken gesichert ist. In der Führungsschiene 10 verläuft eine Spindel, die von einem Motor 35 angetrieben wird. Der Motor 35 weist einen Positionssensor 36 auf, der Informationen über die Spindelposition liefert. Auf der Spindel sitzt eine Spindelmutter, die im Innern des oberen Backens 14 befestigt ist. Durch den Antriebsmotor 35 wird somit der obere Backen 14 in der Höhe verfahren, wobei der Positionssensor 36 eine Information über die Höhenposition liefert.

Der Zwischenbacken 18 ist zusätzlich mit einem Kartuschenträger 37 versehen. Dieser besteht aus einem Ring, in welchen die Kartusche 15 so eingesetzt wird, daß ihr unteres Ende herausragt. Der Kartuschenträger 37 ist seitlich an dem Zwischenbacken 18 angebracht, so daß er das Absenken des oberen Backens 14 auf den Zwischenbacken 18 auch bei eingesetzter Kartusche 15 nicht behindert.

In Fig. 11 ist der Zwischenbacken 18 zur Seite geschwenkt, so daß der Kartuschenträger 37 sich zentrisch über dem Anschluß 24 des unteren Backens 13 befindet. Der untere Nippel der Kartusche 15 ist in den Anschluß 19 eingesetzt und der obere Nippel ist in den Anschluß 20 eingesetzt. Dies ist die Betriebsposition.

Zum Spülen wird zunächst der Motor 35 derart angetrieben, daß er den oberen Backen 14 anhebt. Hierbei wird nach Überschreiten einer bestimmten Position der Zwischenbacken 18 mitgenommen, so daß er den Kartuschenträger 17 hebt und die Kartusche von dem unteren Backen 13 freikommt. Bei weiterem Hochfahren des oberen Bakens 14 wird der Zwischenbacken 18 in die in Fig. 12 dargestellte Ausrichtposition geschwenkt, wobei der Kartuschenträger 37 eine seitliche Position einnimmt. Nach Erreichen der höchsten Stellung des Backens 14 wird der Motor 35 umgeschaltet, so daß er in Gegenrichtung antreibt und den Backen 14 herunterfährt. Beim Herunterfahren drückt der Backen 14 gegen den Zwischenbacken 18, wobei gemäß Fig. 13 alle drei Backen zueinander ausgerichtet und fluidmäßig untereinander verbunden sind. In diesem Zustand kann gespült bzw. desinfiziert werden. Während dies erfolgt, kann die Kartusche 15 aus dem Kartuschenträger 37 entnommen und ausgewechselt werden. Nach Beendigung der Spülung wird der Baken 14 hochgefahren, wobei er nach einer kurzen Hochfahrbewegung den Zwischenbacken 18 verschwenkt und den Kartuschenträger 37 in Ausrichtung mit den beiden äußeren Backen 13 und 14 bringt. Danach wird der obere Backen 14 heruntergefahren, um die Nippel 24,25 der Kartusche in den jeweiligen Sitz des Anschlusses des Backens 13 bzw. 14 zu drücken. Der gesamte Bewegungsablauf wird durch die vom Positionssensor 36 gelieferten Positionssignale gesteuert.

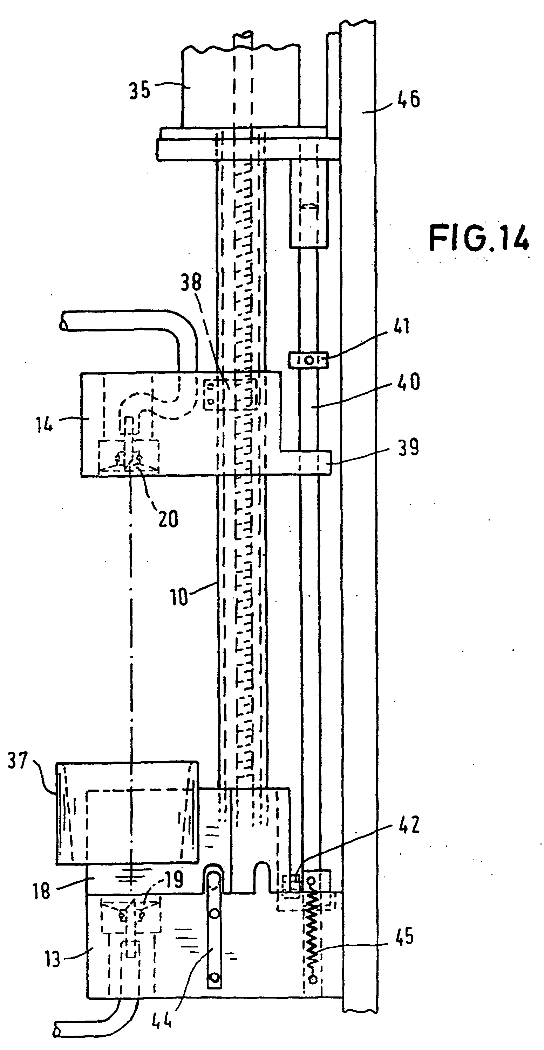

In Fig. 14 ist die Führungsschiene 10 erkennbar, die als Hohlrohr ausgebildet ist und an der Frontseite einen längslaufenden Schlitz aufweist, durch welchen ein Ansatz einer Spindelmutter 38 hindurchragt. Die Spindelmutter, die im Innern der Führungsschiene 10 angeordnet ist, ist an dem oberen Backen 14 befestigt, so daß der Motor 35 die Höhenpositionierung des Backens 14 steuert. Der Backen 14 weist am rückwärtigen Ende einen Ansatz 39 auf, durch den eine Steuerstange 40 hindurchgeht. Diese ist mit einem Mitnahmering 41 versehen. Wenn der Backen 14 hochfährt, nimmt er über den Mitnahmering 41 die Steuerstange 40 mit und diese steuert über einen Nocken 42 die Schwenkbewegung des Zwischenbackens 18. Eine Feder 45 zieht die Steuerstange 40 nach unten. Ein Führungsstift 44 am unteren Backen 13 sorgt dafür, daß der Zwischenbacken 18 nur in exakter Ausrichtung mit dem Backen 13 auf diesen abgesenkt werden kann und garantiert diese Ausrichtung auch beim Hochfahren.

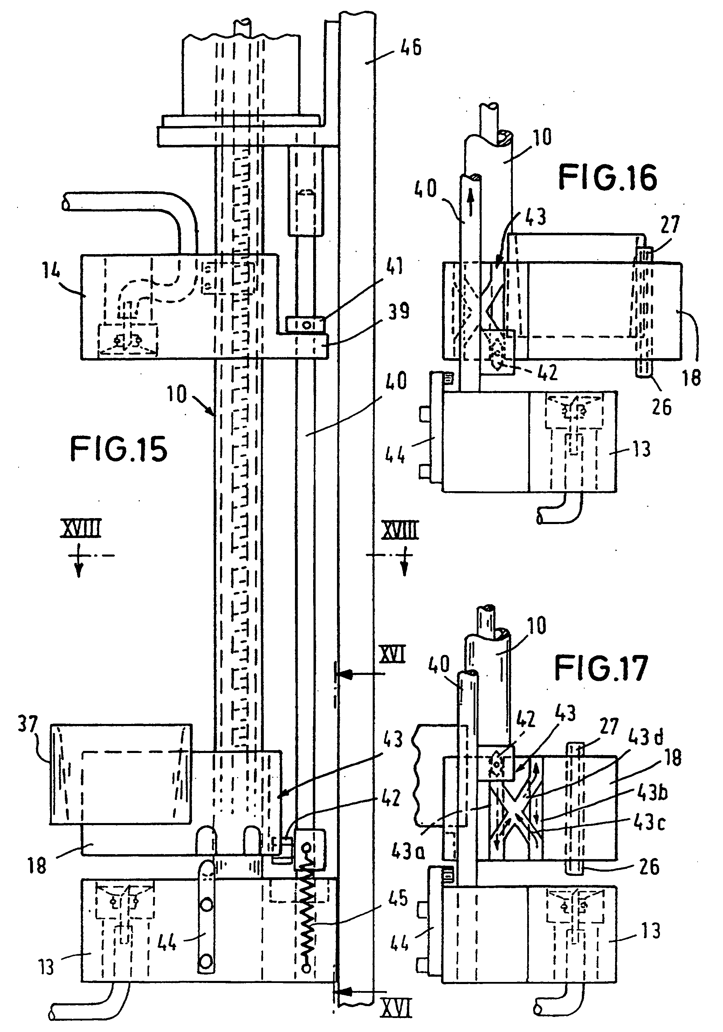

Der Nocken 42 greift in Führungsnuten 43 des Zwischenbackens 18 ein, die in den Fign. 16 und 17 erkennbar sind. Es handelt sich um zwei senkrechte Führungsnuten 43a,43b und zwei diagonale Führungsnuten 43c,43d, welche die senkrechten Führungsnuten verbinden. Bei einer Abwärtsbewegung der Steuerstange 40 fährt der Nocken 42 zunächst in der senkrechten Führungsnut 43a herunter. In der. untersten Endstellung wird der Nocken 42 gering-. fügig gekippt, so daß er beim anschließenden Hochfahren in der schrägen Führungsnut 43d herauffährt und dabei den Zwischenbacken 18 herausschwenkt. Wird dann der Nocken 42 wieder heruntergefahren, so senkt er sich in der Führungsnut 43b ab, wobei der Kartuschenträger 37 abgesenkt und die Kartusche in Position gebracht wird. Beim anschließenden Hochfahren des Nockens 42 läuft dieser in die schräge Führungsbahn 43c, wodurch der Zwischenbacken 18 zunächst angehoben und dann wieder in Ausrichtung mit den beiden äußeren Backen 13,14 gebracht wird.

Die Wand, an der der Kartuschenhalter befestigt ist, ist mit 46 bezeichnet. Dabei handelt es sich beispielsweise um die Vorderwand des Dialysegerätes.

## Patentansprüche

1. Kartuschenhalter für eine Dialysemaschine, mit einem unteren Backen (13) und einem oberen Backen (14), die Anschlüsse für eine Kartusche (15) halten und relativ zueinander bewegbar sind, um eine erste Stellung einzunehmen, in der die Backen (13,14) die Kartusche (15) zwischen sich aufnehmen, und eine zweite Stellung, in der die Backen (13,14) zum Spülen oder Desinfizieren miteinander kurzgeschlossen sind,
**dadurch gekennzeichnet,**
**daß** ein Zwischenbacken (18) vorgesehen ist, der zwischen den unteren und den oberen Backen (13,14) einsetzbar ist, um die Anschlüsse (19,20) dieser Backen zu verbinden.

2. Kartuschenhalter nach Anspruch 1, **dadurch gekennzeichnet, daß** der Zwischenbacken (18) um eine parallel zur Verschieberichtung des oberen oder unteren Backens (13,14) verlaufende Achse schwenkbar ist.

3. Kartuschenhalter nach Anspruch 1 oder 2, **dadurch gekennzeichnet, daß** der Zwischenbacken (18) in Abhängigkeit von der Höhenposition des oberen Backens (14) um eine vertikale Achse schwenkbar ist und dann in die Ebene der beiden äußeren Baken (13,14) gelangt, wenn der obere Backen (14) unter eine Mindesthöhe absinkt.

4. Kartuschenhalter nach einem der Ansprüche 1-3, **dadurch gekennzeichnet, daß** der Zwischenbacken (18) derart gesteuert ist, daß er nach dem Einschwenken in die Ebene der beiden äußeren Backen (13,14) auf den unteren Backen (13) abgesenkt wird.

5. Kartuschenhalter nach einem der Ansprüche 1-4, **dadurch gekennzeichnet, daß** der Zwischenbacken (18) an seiner Unterseite und an seiner Oberseite gleichartige Anschlußelemente (24,25) zum Verbinden mit den ebenfalls untereinander gleichartigen Anschlüssen (19,20) des unteren und des oberen Backens (13,14) aufweist.

6. Kartuschenhalter nach einem der Ansprüche 1-5, **dadurch gekennzeichnet, daß** der Zwischenbacken (18) ein Reservoir zur Aufnahme eines Spül- oder Desinfektionsmittels enthält.

7. Kartuschenhalter nach einem der Ansprüche 1-6, **dadurch gekennzeichnet, daß** der Zwischenbacken (18) einen Kartuschenträger (37) aufweist und derart schwenkbar ist, daß in der einen Position die Anschlußelemente (26,27) und in der anderen Position der Kartuschenträger (37) zu den Anschlüssen (19,20) des unteren und des oberen Backens (13,14) ausgerichtet sind.

8. Kartuschenhalter nach Anspruch 7, **dadurch gekennzeichnet, daß** in der Kurzschlußposition und in der Betriebsposition ein schwenkfreies Absenken des Zwischenbackens (18) in Richtung auf den unteren Backen (13) erfolgt.

## Claims

1. A cartridge holder for a dialyser, comprising a lower jaw (13) and an upper jaw (14), each with connectors for a cartridge (15) and being movable with respect to each other to assume a first position in which the jaws (13, 14) receive the cartridge (15) between them, and a second position in which the jaws (13, 14) are short-circuited for flushing or disinfection,
**characterized in that** an intermediate jaw (18) is provided which is adapted to be inserted between the upper and the lower jaws (13, 14) to interconnect the connectors (19, 20) of these jaws.

2. The cartridge holder of claim 1, wherein the intermediate jaw (18) may be pivoted about an axis extending in parallel to the direction of displacement of the upper or lower jaw (13, 14).

3. The cartridge holder of claim 1 or 2, wherein the intermediate jaw (18) is pivotable about a vertical axis, depending on the vertical position of the upper jaw (14), whereby the intermediate jaw moves into the plane of the two outer jaws (13, 14) when the upper jaw (14) is lowered below a minimum vertical position.

4. The cartridge holder of one of claims 1-3, wherein the intermediate jaw (18) is controlled such that after having pivoted into the plane of the two outer jaws (13, 14), it is lowered onto the lower jaw (13).

5. The cartridge holder of one of claims 1-4, wherein the intermediate jaw (18) has its bottom and top faces provided with similar connection elements (24, 25) for connection with the connectors (19, 20) of the lower and upper jaws (13, 14), which are also similar.

6. The cartridge holder of one of claims 1-5, wherein the intermediate jaw (18) comprises a tank for holding a flushing or disinfecting agent.

7. The cartridge holder of one of claims 1-6, wherein the intermediate jaw ( 18) comprises a cartridge support (37) and is pivotable such that, in the one position, the connection elements (26, 27) and, in the other position, the cartridge support (37) is aligned with the connectors (19, 20) of the lower and upper jaws (13, 14).

8. The cartridge holder of claim 7, wherein in the short-circuit position and in the service position, the intermediate jaw (18) is lowered towards the lower jaw without (13) pivoting.

## Revendications

1. Porte-cartouche pour un appareil de dialyse, comportant une mâchoire inférieure (13) et une mâchoire supérieure (14), qui contiennent les raccordements pour une cartouche (15) et sont mobiles l'une par rapport à l'autre, pour occuper une première position, dans laquelle les mâchoires (13, 14) reçoivent la cartouche (15) entre elles, et une seconde position, dans laquelle les mâchoires (13, 14) sont mises en court-circuit pour le nettoyage ou la désinfection,
**caractérisé en ce**
**qu'**une mâchoire intermédiaire (18) est prévue, qui peut être insérée entre la mâchoire supérieure et la mâchoire inférieure (13, 14), afin de relier les raccordements (19, 20) de cette mâchoire.

2. Porte-cartouche selon la revendication 1, **caractérisé en ce que** la mâchoire intermédiaire (18) peut pivoter autour d'un axe parallèle à la direction de déplacement de la mâchoire (13, 14) inférieure ou supérieure.

3. Porte-cartouche selon la revendication 1 ou 2, **caractérisé en ce que** la mâchoire intermédiaire (18) peut pivoter autour d'un axe vertical en fonction de la position de hauteur de la mâchoire supérieure (14) et arrive ensuite au niveau des deux mâchoires extérieures (13, 14), lorsque la mâchoire supérieure (14) descend au-dessous d'une hauteur minimum.

4. Porte-cartouche selon l'une quelconque des revendications 1 à 3, **caractérisé en ce que** la mâchoire intermédiaire (18) est actionné de sorte qu'elle soit descendue sur la mâchoire inférieure (13) après qu'elle a été placée par pivotement au niveau des deux mâchoires extérieures (13, 14).

5. Porte-cartouche selon l'une quelconque des revendications 1 à 4, **caractérisé en ce que** la mâchoire intermédiaire (18) présente sur sa face inférieure et sur sa face supérieure des éléments de raccordement (24, 25) de même nature pour les relier aux raccordements (19, 20) également de même nature de la mâchoire (13, 14) inférieure et supérieure.

6. Porte-cartouche selon l'une quelconque des revendications 1 à 5, **caractérisé en ce que** la mâchoire intermédiaire (18) contient un réservoir pour recevoir un moyen de nettoyage ou de désinfection.

7. Porte-cartouche selon l'une quelconque des revendications 1 à 6, **caractérisé en ce que** la mâchoire intermédiaire (18) présente un porte-cartouche (37) et peut pivoter de sorte que, dans une position, les éléments de raccordement (26, 27) et, dans l'autre position, le porte-cartouche (37) soient alignés sur les raccordements (19, 20) des mâchoires (13, 14) inférieure et supérieure.

8. Porte-cartouche selon la revendication 7, **caractérisé en ce que** dans la position de mise en court-circuit et dans la position de service, une descente de la mâchoire intermédiaire (18) en direction de la mâchoire inférieure (13) est effectuée sans pivotement.
